# EUROPEAN PATENT SPECIFICATION

(11) **EP 4 358 829 B1**
(45) Date of publication and mention of the grant of the patent: **14.10.2026**
(21) Application number: 22827852.9
(22) Date of filing: 22.06.2022
(51) Int. Cl.: A61B 5/00, A61B 5/055

(54) **METHOD, COMPUTER SOFTWARE PRODUCT AND SYSTEM FOR DETERMINING CONDITION OF A SUBJECT BASED ON CONNECTOME**
VERFAHREN, COMPUTERPROGRAMM-PRODUKT UND SYSTEM ZUR BESTIMMUNG DES ZUSTANDS EINER PERSON AUF DER BASIS EINES KONNEKTIVITÄTS
PROCÉDÉ, PRODUIT LOGICIEL ET SYSTÈME POUR DÉTERMINER L'ÉTAT D'UN SUJET SUR LA BASE D'UN CONNECTOME

(30) Priority: 22.06.2021 US 202163213242 P
(43) Date of publication of application: 01.05.2024
(73) Proprietor: Sheba Impact Ltd., 5265601 Ramat-Gan (IL)
(72) Inventor: LIVNY-EZER, Abigail, 6949403 Tel-Aviv (IL); MENINGHER, Inbar, 6772952 Tel-Aviv (IL); BERLINERBLAU, Gregory, 7552678 Rishon LeTsion (IL); RAIZMAN, Reut Moran, 4840902 Rosh HaAyin (IL)
(74) Representative: J A Kemp LLP
(86) International application number: PCT/IL2022/050671
(87) International publication number: WO 2022/269613

(56) References cited:
- GORKA ZAMORA-L\'OPEZ ET AL: "Functional complexity emerging from anatomical constraints in the brain: the significance of network modularity and rich-clubs", ARXIV.ORG, CORNELL UNIVERSITY LIBRARY, 201 OLIN LIBRARY CORNELL UNIVERSITY ITHACA, NY 14853, 24 February 2016 (2016-02-24), XP080685754
- WANG YAN ET AL: "A Novel Multimodal MRI Analysis for Alzheimer's Disease Based on Convolutional Neural Network", 2018 40TH ANNUAL INTERNATIONAL CONFERENCE OF THE IEEE ENGINEERING IN MEDICINE AND BIOLOGY SOCIETY (EMBC), IEEE, 18 July 2018 (2018-07-18), pages 754 - 757, XP033431819, DOI: 10.1109/EMBC.2018.8512372
- TYLER M SEIBERT ET AL: "Default network correlations analyzed on native surfaces", JOURNAL OF NEUROSCIENCE METHODS, ELSEVIER SCIENCE PUBLISHER B.V., AMSTERDAM, NL, vol. 198, no. 2, 7 April 2011 (2011-04-07), pages 301 - 311, XP028387538, ISSN: 0165-0270, [retrieved on 20110414], DOI: 10.1016/J.JNEUMETH.2011.04.010
- YAAKUB SITI NURBAYA ET AL: "On brain atlas choice and automatic segmentation methods: a comparison of MAPER & FreeSurfer using three atlas databases", SCIENTIFIC REPORTS, vol. 10, no. 1, 18 February 2020 (2020-02-18), US, XP093337338, ISSN: 2045-2322, Retrieved from the Internet <URL:https://www.nature.com/articles/s41598-020-57951-6> DOI: 10.1038/s41598-020-57951-6
- SARRAF SAMAN ET AL: "DeepAD: Alzheimer's Disease Classification via Deep Convolutional Neural Networks using MRI and fMRI", BIORXIV, 14 January 2017 (2017-01-14), pages 1 - 32, XP093338035, Retrieved from the Internet <URL:https://www.biorxiv.org/content/10.1101/070441v4.full.pdf> DOI: 10.1101/070441
- ZHANG LU; WANG LI; GAO JEAN; RISACHER SHANNON L.; YAN JINGWEN; LI GANG; LIU TIANMING; ZHU DAJIANG: "Deep Fusion of Brain Structure-Function in Mild Cognitive Impairment", MEDICAL IMAGE ANALYSIS, OXFORD UNIVERSITY PRESS, OXOFRD, GB, vol. 72, 23 April 2021 (2021-04-23), GB , XP086702585, ISSN: 1361-8415, DOI: 10.1016/j.media.2021.102082

## Description

### RELATED APPLICATION

This application claims the benefit of priority of U.S. Provisional Patent Application No. 63/213,242 filed on 22 June 2021.

### FIELD AND BACKGROUND OF THE INVENTION

The present invention, in some embodiments thereof, relates to neurology and psychiatry and, more particularly, but not exclusively, to a method and system for determining a condition of a subject based on connectome. Some embodiments of the present invention relates to a method and system for predicting outcome of the condition, and/or a response to a treatment.

Magnetic Resonance Imaging (MRI) is a method to obtain an image representing the chemical and physical microscopic properties of materials, by utilizing a quantum mechanical phenomenon, named Nuclear Magnetic Resonance (NMR), in which a system of spins, placed in a magnetic field resonantly absorbs energy, when applied with a certain frequency. In MRI, a static magnetic field having a gradient is applied on an object, thereby creating, at each depth of the object, a unique magnetic field. By detecting the NMR signal, knowing the magnetic field gradient, the position of each region of the object can be imaged.

Functional MRI (fMRI) is an MRI technique that locates activated brain regions by detecting, via the aforementioned NMR phenomenon, differences between a state of the hemoglobin in which oxygen is bound thereto (oxygenated hemoglobin) and a state of the hemoglobin in which oxygen released therefrom (deoxygenated hemoglobin). The signal differentiating between these two hemoglobin states is referred to as blood-oxygenation-level-dependent (BOLD) signal. It is considered that BOLD signals are increased in an active region.

Known in the art is an MRI technique called diffusion weighted imaging (DWI). In DWI, a pulse sequence is selected to allow acquisition of an image signal that is dependent upon the diffusivity of the tissue. One example implementation of DWI is Diffusion Tensor Imaging (DTI), which is an MRI technique that captures orientations of the axonal bundles, and that uses the DWI pulse sequence to apply motion sensitizing magnetic field gradients so that the magnetic resonance (MR) images include contrast related to the diffusion of nuclear spins. By applying the diffusion gradients in selected directions, diffusion constant of water molecules are measured along multiple orientations, and apparent diffusion tensor coefficients are obtained for each voxel location in the image. Fluid molecules diffuse more readily along the direction of the axonal fiber bundle as compared with other directions. Hence, the directionality and anisotropy of the apparent diffusion coefficients correspond with the direction of the axonal fibers and fiber bundles.

A map of neural connections in the brain is referred to as a connectome. In graph theory terms, a connectome consists of the set of neural elements (nodes) and their interconnections (edges), where the nodes typically represent brain regions, and the edges typically represent connections. The connections can be structural, in which case the connectome is referred to as a structural connectome (SC), or functional, in which case the connectome is referred to as a functional connectome (FC). A SC expresses the connection among brain regions by axonal fibers and is therefore conventionally constructed by analysis of data obtained by DTI. FC expresses the correlation among activated brain regions and is therefore conventionally constructed by analysis of data obtained by fMRI.

The article by Zhang Lu et al: "Deep Fusion of Brain Structure-Function in Mild Cognitive Impairment", MEDICAL IMAGE ANALYSIS, OXFORD UNIVERSITY PRESS, OXOFRD, GB, vol. 72, 23 April 2021, relates to a graph-based deep neural network to simultaneously model brain structure and function.

### SUMMARY OF THE INVENTION

According to the present invention there is provided a method of determining a condition of a subject. The method comprises receiving functional magnetic resonance (MR) data and structural MR data, each describing the brain of the subject; constructing a subject-specific functional connectome (FC) using the functional MR data, and a subject-specific structural connectome (SC) using the structural MR data; accessing a computer readable medium storing a trained convolutional neural network (CNN) having a first set of layers trained for separately processing FC and SC, and a second set of layers trained for processing combined outputs from the first set of layers; feeding the CNN with the subject-specific FC and SC; and receiving from the second set of layers an output indicative of a condition of the subject.

According to the invention the method comprises defining a parcellation of the brain using an anatomical atlas defined over a standardized space.

According to the invention the functional MR data and the structural MR data describe the brain in a respective native space of the brain, and wherein the parcellation comprises applying a transformation of images of the atlas onto the respective native space, to provide respective parcellated functional MR data and parcellated structural MR data over the respective native space.

According to some embodiments of the invention the method comprises calculating the transformation by receiving a mean MR image of the brain, and registering a template image defined over the standardized space onto the mean magnetic resonance image.

According to some embodiments of the invention the mean MR image is based on a volume average of at least one of the structural and functional MR data.

According to some embodiments of the invention the separate processing comprise generating a plurality of FC activation maps and a plurality of SC activation maps, and wherein the combined outputs comprise a concatenation between a respective FC activation map and respective SC activation map.

According to the invention the first set of layers comprises one hidden convolutional layer trained for separately processing FC, and one hidden convolutional layer trained for separately processing SC.

According to some embodiments of the invention the first set of layers comprises more than one hidden convolutional layer trained for separately processing FC.

According to some embodiments of the invention the first set of layers comprises more than one hidden convolutional layer trained for separately processing SC.

According to some embodiments of the invention the second set of layers comprises at least two hidden convolutional layers.

According to the invention the subject-specific FC is constructed at least by extracting from the functional MR data a plurality of time-ordered series of values, each series corresponding to a different region of the brain, and constructing a correlation matrix describing correlation among the plurality of series, wherein the subject-specific FC is the correlation matrix.

According to some embodiments of the invention the correlation is selected from the group consisting of a pairwise correlation, a partial correlation, and a distance correlation.

According to some embodiments of the invention the subject-specific SC is constructed at least by applying whole brain tractography to define a plurality of streamlines between pairs of regions of the brain, and converting the plurality of streamlines to a connectivity matrix, wherein the subject-specific SC is the connectivity matrix.

According to some embodiments of the invention the CNN is further trained for predicting a response to a treatment for the condition.

According to some embodiments of the invention the CNN is further trained for predicting a clinical outcome of the condition.

According to some embodiments of the invention the CNN is trained for predicting a likelihood for at least one condition selected from the group consisting of brain concussion, depressive disorder, stroke, traumatic brain injury, post-traumatic stress disorder, epilepsy, Parkinson, multiple sclerosis, agitation, abuse, Alzheimer's disease, anxiety, panic, phobic disorder, bipolar disorder, borderline personality disorder, behavior control disorder, body dysmorphic disorder, cognitive impairment, dissociative disorder, eating disorder, fatigue, impulse-control disorder, irritability, obsessive-compulsive disorder, personality disorder, psychotic disorder, sexual disorder, sleep disorder, stuttering, Tourette's Syndrome, Trichotillomania, self-destructive behavior, fibromyalgia, tremor, schizophrenia, attention-deficit disorder, hyperactivity disorder, and learning disorder.

According to an aspect of some embodiments of the present disclosure there is provided a method of treating a disorder. The method comprises executing the method as delineated above and optionally and preferably as further detailed below to determine a disorder for the subject; and applying to the subject a treatment selected to specifically treat the determined disorder.

According to an aspect of some embodiments of the present invention there is provided a computer software product. The product comprises a computer-readable medium in which program instructions are stored, which instructions, when read by an image processor, cause the data processor to receive functional MR data and structural MR data, each describing the brain of a subject, and execute the method as delineated above and optionally and preferably as further detailed below.

According to an aspect of some embodiments of the present invention there is provided a magnetic resonance imaging (MRI) system for imaging a brain of a subject. The system comprises an MRI scanner configured for scanning the brain to provide functional MR data and structural MR data, each describing the brain; and an image processor configured for executing the method as delineated above and optionally and preferably as further detailed below.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE SEVERAL VIEWS OF THE DRAWINGS

Some embodiments of the invention are herein described, by way of example only, with reference to the accompanying drawings and images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart diagram of a method suitable for determining a condition of a subject, according to some exemplary embodiments of the present invention;
FIG. 2 is a schematic illustration of a representative example for a technique suitable for constructing a subject-specific functional connectome, according to some embodiments of the present invention;
FIGs. 3A and 3B are schematic illustrations of a representative example for a technique suitable for constructing a subject-specific structural connectome, according to some embodiments of the present invention;
FIG. 4 is a schematic illustration of a representative example of a trained convolutional neural network (CNN), according to some embodiments of the present invention;
FIG. 5 is a schematic illustration of an MRI system for imaging a body, according to some embodiments of the present invention;
FIG. 6 shows image processing techniques for normalization (top panel) and personalization (bottom panel) of MR images, according to some embodiments of the present invention; and
FIG. 7 is a schematic illustration of a CNN architecture employed in experiments performed according to some embodiments of the present invention.

### DESCRIPTION OF SPECIFIC EMBODIMENTS OF THE INVENTION

The present invention, in some embodiments thereof, relates to neurology and psychiatry and, more particularly, but not exclusively, to a method and system for determining a condition based on connectome. Some embodiments of the present invention relates to a method and system for predicting outcome of the condition, and/or a response to, a treatment.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways. The scope of the invention is defined by the claims.

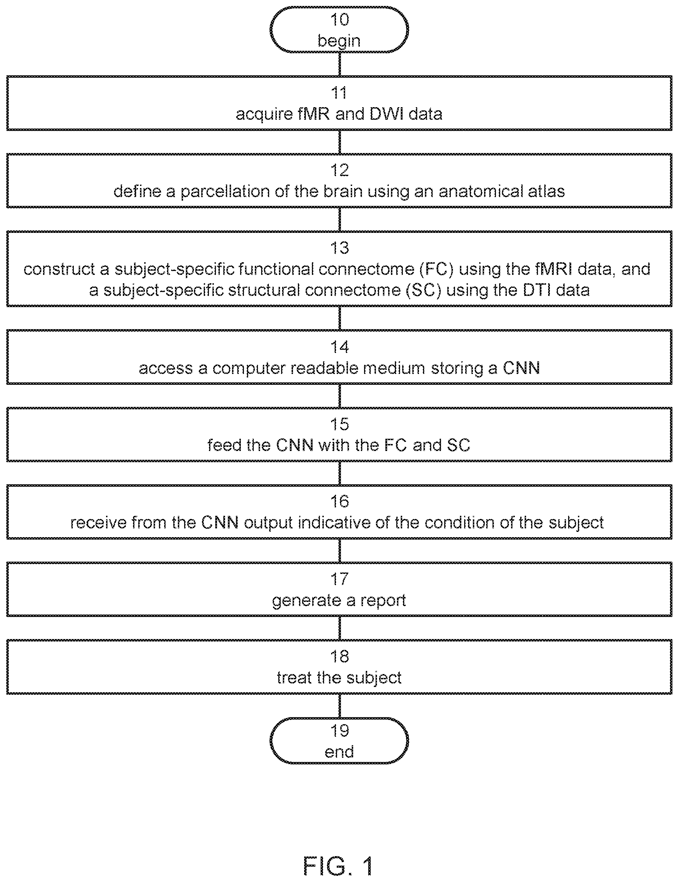

FIG. 1 is a flowchart diagram of a method suitable for determining a condition of a subject, preferably a mammalian subject, *e.g.,* a human subject, according to various exemplary embodiments of the present invention. It is to be understood that, unless otherwise defined, the operations described herein below can be executed either contemporaneously or sequentially in many combinations or orders of execution. Specifically, the ordering of the flowchart diagrams is not to be considered as limiting. For example, two or more operations, appearing in the following description or in the flowchart diagrams in a particular order, can be executed in a different order (e.g., a reverse order) or substantially contemporaneously. Additionally, several operations described below are optional and may not be executed.

The condition to be determined by the method can be a neurological condition and/or a mental (*e.g.*, psychiatric) condition.

At least part of the operations described herein can be implemented by a data processing system, *e.g.,* a dedicated circuitry or a general purpose image processor, configured for receiving magnetic resonance (MR) data and executing the operations described below. At least part of the operations can be implemented by a cloud-computing facility at a remote location.

Computer programs implementing the method of the present embodiments can commonly be distributed to users by a communication network or on a distribution medium such as, but not limited to, a floppy disk, a CD-ROM, a flash memory device and a portable hard drive. From the communication network or distribution medium, the computer programs can be copied to a hard disk or a similar intermediate storage medium. In some embodiments, the method executes computer instructions of a docker that contains instructions to receive MR data and instructions to process the MR data, wherein the instructions to process the MR data include a set of instructions to generate a connectome and a set of instructions to apply machine learning. Optionally and preferably, the docker's instructions to process the MR data are executed automatically, without user intervention. The computer programs can be run by loading the code instructions either from their distribution medium or their intermediate storage medium into the execution memory of the computer, configuring the computer to act in accordance with the method of this invention. During operation, the computer can store in a memory data structures or values obtained by intermediate calculations and pull these data structures or values for use in subsequent operation. All these operations are well-known to those skilled in the art of computer systems.

Processing operations described herein may be performed by means of processer circuit, such as a DSP, microcontroller, FPGA, ASIC, etc., or any other conventional and/or dedicated computing system.

The method of the present embodiments can be embodied in many forms. For example, it can be embodied in on a tangible medium such as a computer for performing the method operations. It can be embodied on a computer readable medium, comprising computer readable instructions for carrying out the method operations. In can also be embodied in electronic device having digital computer capabilities arranged to run the computer program on the tangible medium or execute the instruction on a computer readable medium.

The method begins at **10** and optionally and preferably continues to **11** at which MR data are acquired from the brain of the subject. The MR data can be acquired by an MR scanner having a controller configured for generating a pulse sequence suitable for acquiring an MR signal from which functional MR data can be obtained, and also a pulse sequence suitable for acquiring an MR signal from which structural MR data can be obtained.

Representative examples of functional MR data suitable for the present embodiments include, without limitation, fMRI data, and arterial spin labeling data.

Representative examples of structural MR data suitable for the present embodiments include, without limitation, diffusion weighted imaging scan data (DWI, *e.g.*, multi-shell, diffusion kurtosis imaging), and T1-weighted data.

In some embodiments of the present invention the functional MR data comprise fMRI data, and in some embodiments of the present invention the structural MR data comprise DWI data.

While the embodiments below are described with a particular emphasis to cases in which the functional MR data are fMRI data, and the structural MR data are DWI data, it is to be understood that use of other types of functional MR data and/or other types of structural MR data are also contemplated.

When the functional MR data comprise fMRI data, and the structural MR data comprise DWI data, the controller of the scanner can generate a T₂*-weighted gradient echo or T₂-weighted spin echo pulse sequence so as to induce a BOLD signal from which the fMRI data can be obtained, and a diffusion-weighted pulse sequence, which preferably employs more than three (*e.g.,* six or more, more preferably sixteen or more) gradient directions, from which the DWI data can be obtained.

In some embodiments of the present invention operation **11** also includes acquisition of MR data for use as a registration MR image of the brain in the common practice preprocessing steps of both fMRI and DWI data. The registration MR image can also be used for parcellation of the MR data, particularly of the fMRI data, but may also be used for parcellation of the DWI data. The registration MR image can be acquired by applying a structural pulse sequence to the brain, such as, but not limited to, a T₁-weighted pulse sequence, a T₂-weighted pulse sequence, and a proton density-weighted pulse sequence. In experiments performed by the Inventors, the registration MR image was a T₁-weighted MR image acquired by applying to the brain a T₁-weighted pulse sequence. The advantage of acquiring a T₁-weighted MR image as the registration image is that the T₁-weighted scan outputs a contrast between the different brain tissues i.e. grey-matter, white-matter and cerebrospinal fluid, and can therefore be used to provide a more accurate brain parcellation.

In some embodiments of the present invention all the MR data are received by the method from an external source, *e.g.,* a computer readable medium or a cloud storage facility, in which case operation **11** can be skipped. Also contemplated are embodiments in which some of the MR data are received by the method from an external source.

The method continues to **12** at which a parcellation of the MR data of the brain is executed using an anatomical atlas. The anatomical atlas defines a plurality of brain regions, including cortical regions, subcortical regions, and cerebellar regions. Preferably, the anatomical atlas defines at least 50 or at least 100 or at least 150 or at least 200 or at least 250 brains regions. A preferred anatomical atlas suitable for the present embodiments is the Brainnetome atlas [Fan et al., 2016, Cerebral Cortex, Volume 26, Issue 8, Pages 3508-3526], but other anatomical atlases, such as, but not limited to, the Automated anatomical labeling atlas [Rolls et al., 2020, NeuroImage, Volume 206, 116189, ISSN 1053-8119], and the Desikan-Killiany atlas [Desikan et al., 2006, NeuroImage, Volume 31, Issue 3, Pages 968-980] are also contemplated.

The anatomical atlas is typically defined in a standardized space, for example, the Montreal Neurological Institute (MNI) space. On the other hand, the MR data are typically defined in a native space of the subject, which can be the same native space for both fMRI and DWI data, or, alternatively, the native spaces for fMRI data and DWI data can differ. Thus, the parcellation **12** of the brain typically comprises applying a transformation between the subject's respective native space and the standardized space. The transformation can be from the native space of the subject to the standardized space, as typically done traditionally in a process known as normalization, or, more preferably, from the standardized space to the native space of the subject.

As demonstrated in the Examples section that follows, the Inventors surprisingly found that better classification results are obtained when applying a personalization process including a transformation from the standardized space to the native space of the subject, and performing the parcellation **12** in the native space of the subject.

In the present invention the personalization is conducted by a transformation of the atlas into a native space image. fMRI data can be processed to obtain a skull stripped averaged image of all the resting-state scan volumes, and the DWI data can be processed to obtain a skull-stripped averaged image of all the diffusion-weighted volumes. Each of those averaged images can enact the native space image for the respective native space.

A representative example of a parcellation according to some embodiments of the present invention is as follows. First, a transformation of a template image or of a parceled and segmented T1 image from the standardized space (e.g., MNI template image) to the native space image is conducted. This transformation warps the template image or the parceled and segmented T1 image by applying a series of 3D geometric operations, including, without limitation, translation, rotation, scaling, and shearing. The series of 3D geometric operations is recorded as a transformation matrix, which is then applied to the atlas image, thus defining a personalized atlas image containing a parcellation of the brain in the native space of the subject.

The method proceeds to **13** at which a subject-specific functional connectome (FC) is constructed using the fMRI data, and a subject-specific structural connectome (SC) is constructed using the DWI data.

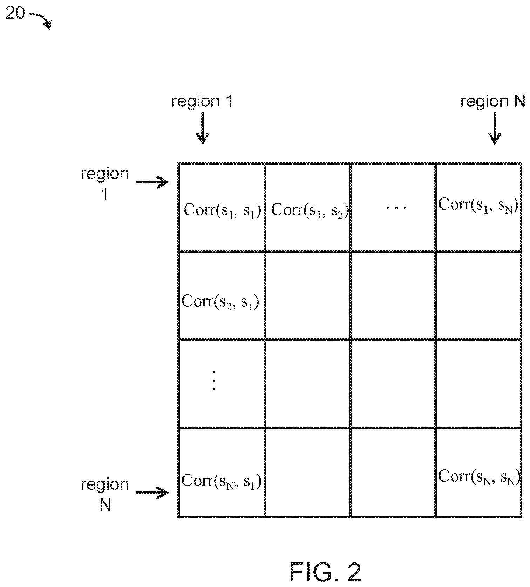

The FC can be constructed by any technique known in the art. A representative example for a technique suitable for constructing a subject-specific FC, will now be described with reference to FIG. 2.

The FC is constructed according to the present invention by extracting from the fMRI data a plurality of time-ordered series of values, one series for each region of the brain. The regions can be the regions that were defined at the parcellation **12**. Thus, labeling the brain regions numerically from 1 to N, the method can extract for the ith region (i=1,...,N) a time-ordered series *s*i={*b*ᵢ(*t=t*₁), *b*ᵢ(*t=t*₂), *..., b*ᵢ(*t=t*_{T})} where t₁<t₂<...<*t*_{T} is a set of time points, and where each element of sᵢ is a value that represents the BOLD signal acquired from the ith region at the respective time point. For example, each element of sᵢ can be a spatial average of the BOLD values over the ith region. A similar time-ordered series is extracted from the fMRI data for each of the other regions of the brain. Once the series *s*₁, *s*₂, ..., *s*_{N} are extracted from the fMRI data, the method calculates pairwise correlations among the series and constructs a correlation matrix describing these pairwise correlations. FIG. 2 illustrates a representative example of such a correlation matrix **20**.

Matrix **20** is a square N×N matrix of which the (*i*,*j*) element contains a value of correlation function Corr(sᵢ, sⱼ) between the series sᵢ and the series sⱼ. The correlation function Corr(sᵢ, sⱼ) is optionally and preferably symmetric such that Corr(sᵢ, sⱼ) = Corr(sⱼ, sᵢ), and Corr(sᵢ, sᵢ) represents the perfect correlation. In experiments performed by the Inventors Corr(sᵢ, sⱼ) was implemented as a function that calculates the Pearson's correlation coefficient, but other types of correlation functions, such as, but not limited to, a function that calculates distance correlation, are also contemplated according to some embodiments of the present invention. Once matrix **20** is constructed it can be defined as the FC.

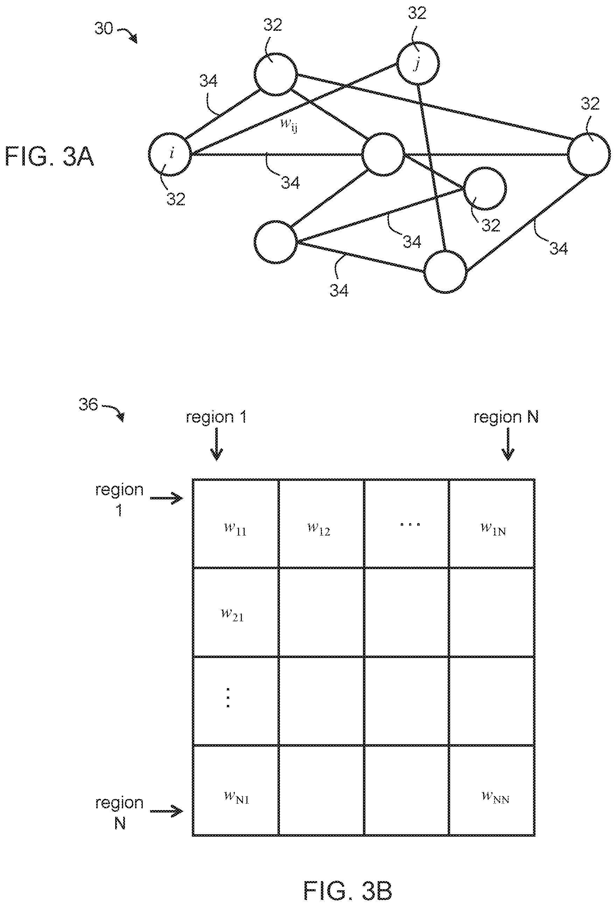

The SC can also be constructed by any technique known in the art. A representative example for a technique suitable for constructing a subject-specific SC, will now be described with reference to FIGs. 3A and 3B.

For example, a deterministic or probabilistic tractography can be employed. In these embodiments, each voxel encompasses a main diffusion direction value. Then, a streamline can elongate from one voxel to the next, optionally and preferably under 2 restrictions: white matter integrity values over a predetermined integrity value threshold (e.g., about 0.1, or about 0.15, or about 0.2 or about 0.25) and an angle of over a predetermined angle threshold (e.g., about 35 or about 40 or about 45 or about 50 degrees) between the main diffusion direction of both voxels. This process is repeated until an entire pathway is traced, outputting a whole-brain tractography map. In some embodiments of the present invention whole-brain tractography is employed to define a plurality of streamlines between pairs of regions of the brain. The regions can be the regions that were defined at the parcellation **12.**

With reference to FIG. 3A, once the whole-brain tractography is completed, a weighted non-directed graph **30** with a plurality of nodes **32** and a plurality of edges **34** can be constructed. Each node **32** can represent a region of the brain, and each edge **34** can be assigned with a weight value. FIG. 3A explicitly illustrates an exemplified weight value *w*ᵢⱼ between the ith node (representing region *i* of the brain) and the jth node (representing region *j* of the brain). In some embodiments of the present invention the weight value of an edge is selected based on the number of streamlines or fractional anisotropy values (FA) that are found during the whole-brain tractography between the regions that correspond to the pair of nodes connected by this edge. For example, weight value *w*ᵢⱼ is selected based on the number of streamlines between regions *i* and *j* of the brain. Preferably the weight value is selected based only on the respective number of streamlines, so that, for example, *w*ᵢⱼ = *w*ⱼᵢ. In some embodiments of the present invention a thresholding operation is applied to the number of streamlines, before assigning the weights. For example, when the number of streamlines is below a predetermined threshold, the respective weight value can be set to zero.

With reference to FIG. 3B, once the non-directed graph **30** is constructed, a connectivity matrix **36** can be constructed. Connectivity matrix **36** can be similar to correlation matrix **20,** except that the matrix-elements of connectivity matrix **36** are the weight values of the edges of graph **30,** rather than the aforementioned correlation function. An exception are the matrix-elements on the main diagonal of connectivity matrix **36** (*w*₁₁, *w*₂₂, ..., *w*_{NN}), which are optionally and preferably selected according to a separate criterion, as a region may have more than one connection to itself, and therefore different regions may have different respective values on the diagonal.

Referring, again to FIG. 1, the method continues to **14** at which a computer readable medium storing a trained convolutional neural network (CNN) is accessed.

Artificial neural networks are a class of machine learning procedures based on a concept of inter-connected computer program objects referred to as neurons. In a typical artificial neural network, neurons contain data values, each of which affects the value of a connected neuron according to a pre-defined weight (also referred to as the "connection strength"), and whether the sum of connections to each particular neuron meets a pre-defined threshold. By determining proper connection strengths and threshold values (a process also referred to as training), an artificial neural network can achieve efficient recognition of patterns in data. Oftentimes, these neurons are grouped into layers. Each layer of the network may have differing numbers of neurons, and these may or may not be related to particular qualities of the input data. An artificial neural network having an architecture of multiple layer belongs to a class of artificial neural networks referred to as deep neural network.

In one implementation, called a fully-connected network, each of the neurons in a particular layer is connected to and provides input values to each of the neurons in the next layer. These input values are then summed and this sum is used as an input for an activation function (such as, but not limited to, ReLU or Sigmoid). The output of the activation function is then used as an input for the next layer of neurons. This computation continues through the various layers of the neural network, until it reaches a final layer. At this point, the output of the fully-connected network can be read from the values in the final layer.

Convolutional neural networks (CNNs) include one or more convolutional layers in which the transformation of a neuron value for the subsequent layer is generated by a convolution operation. The convolution operation includes applying a convolutional kernel (also referred to in the literature as a filter) multiple times, each time to a different patch of neurons within the layer. The kernel typically slides across the layer until all patch combinations are visited by the kernel. The output provided by the application of the kernel is referred to as an activation map of the layer. Some convolutional layers are associated with more than one kernel. In these cases, each kernel is applied separately, and the convolutional layer is said to provide a stack of activation maps, one activation map for each kernel. Such a stack is oftentimes described mathematically as an object having D+1 dimensions, where D is the number of lateral dimensions of each of the activation maps. The additional dimension is oftentimes referred to as the depth of the convolutional layer. For example, in CNNs that are configured to process two-dimensional image data, a convolutional layer that receives the two-dimensional image data provides a three-dimensional output, with two-dimensional activation maps and one depth dimension.

The advantage of using CNN is the use of layers of kernels providing the ability to learn different levels of complexity of visual aspects of the input features.

A CNN can be trained according to some embodiments of the present invention by feeding a CNN training program with training data. The training data includes labeled pairs of connectomes obtained from a cohort of subjects. The connectomes of the training data include both FCs and SCs that are labeled as characterizing subjects that have been diagnosed with a condition (e.g., neurological condition, psychiatric condition), and FCs and SCs that are labeled as characterizing control subjects (*e.g.*, healthy subjects, or subjects that do not present a neurological and/or psychiatric condition). In some embodiments of the present invention the connectomes of the training data include FCs and SCs that are labeled as characterizing subjects with positive or negative treatment response, and in some embodiments of the present invention the connectomes of the training data include FCs and SCs that are labeled with clinical outcome measures. The training process adjusts convolutional kernels, bias matrices and other parameters of the CNN so as to produce an output that classifies and/or predicts clinical outcome for each pair of FC and SC as close as possible to its label. The final result of the training is a trained CNN having an input layer, at least one, more preferably a plurality of, hidden layers, and an output layer, with adjusted weights assigned to each component (neuron, layer, kernel, etc.) of the network. Once the training data are fed, the CNN training program generates a trained CNN which can then be used without the need to re-train it.

Following the training, a validation process may optionally and preferably be applied to the trained CNN, by feeding validation data into the network. The validation data is typically of similar type as the training data, except that only the FCs and SCs are fed to the trained network, but not their labels. The labels are used for validation by comparing the output of the trained CNN to the labels.

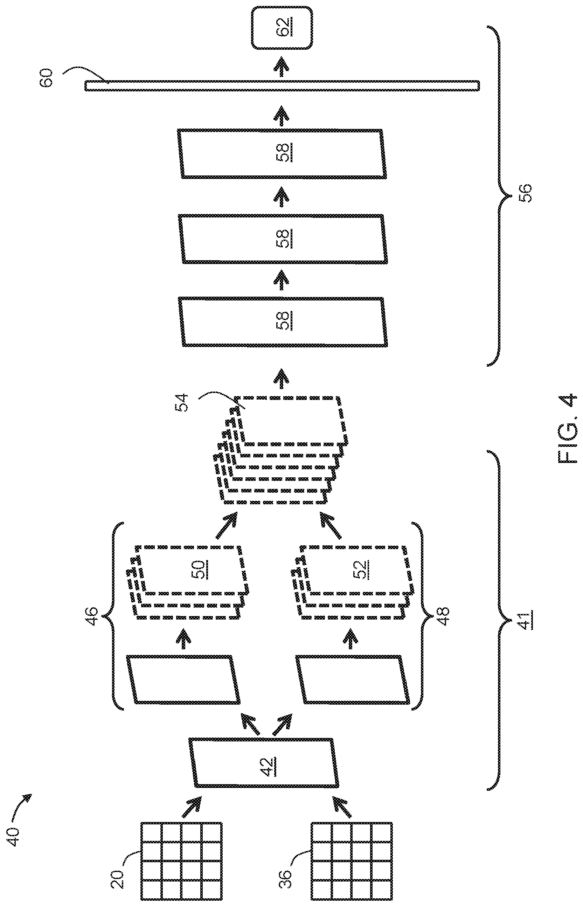

A representative example of a trained CNN **40**, according to some embodiments of the present invention is schematically illustrated in FIG. 4. CNN **40** preferably comprises a set of layers **41** trained for receiving and processing FC **20** and SC **36**, separately. Set **41** comprises an input layer **42** configured to receive two-dimensional input containing FC **20** and SC **36**. FIG.4 illustrates an embodiment in which the same input layer **42** receives both FC **20** and SC **36**. Also contemplated, are embodiments in which set **41** comprises a pair of input layers (not shown), one input layer that receives FC **20** and one input layer that receives SC **36**. Set **41** can also comprise a pair of convolutional layers **46**, **48**, respectively trained for processing FC **20** and SC **36,** separately. Layers **46** and **48** are preferably hidden layers, but the present embodiments also contemplate an architecture in which layers **46** and **48** also comprise input layers, in which case there is no need for layer **42.** Each of the layers **46** and **48** is optionally and preferably associated with a set of kernels. The kernels are preferably row kernels. As demonstrated in the Examples section that follows, the inventors found that row kernels provide higher classification accuracy. However, use of square kernels is also contemplated in some embodiments of the invention.

Each of layers **46** and **48** is two-dimensional, and optionally and preferably produces a three-dimensional output **50** and **52**, respectively, where the number of two-dimensional activation maps in each of outputs **50** and **52** equals the number of kernels associated with layers **46** and **48.** In some embodiments of the present at least one of layers **46** and **48** is associated with a single kernel, in which case the respective output (**50** and/or **52**) includes only one activation map, and can therefore be viewed as a two-dimensional output. In some embodiments of the present a row kernel is used, and applied thus reducing the dimension of the output activation map associated with each kernel, wherein the dimension of the output activation map of the respective layer is increased to two dimensions by stacking the outputs resulting from two or more such row kernels, thus also obtaining a two-dimensional output.

Optionally and preferably, layers **46** and **48** also apply a nonlinear activation function, to each of the produced activation maps.

Many types of nonlinear activation functions that are known in the art, can be used in CNN **40**, including, without limitation, Rectified linear unit (ReLU), Leaky rectified linear unit, Parameteric rectified linear unit (PReLU), Randomized leaky rectified linear unit (RReLU), Exponential linear unit (ELU), Scaled exponential linear unit (SELU), S-shaped rectified linear activation unit (SReLU), Inverse square root linear unit (ISRLU), Adaptive piecewise linear (APL), SoftPlus, Bent identity, SoftExponential, Sinusoid, Sinc, Gaussian, Softmax, Binary step, Soft step, TanH, ArcTan, Softsign, Inverse square root unit (ISRU), and Maxout. In some embodiments of the present invention a ReLU activation function or a variant thereof (*e.g.,* PReLU, RReLU, SReLU) is used.

In some embodiments of the present invention set **41** comprises a single hidden convolutional layer **46** trained for separately processing FC **20**, and a single hidden convolutional layer **48** trained for separately processing SC **36.** In some embodiments of the present invention CNN **40** does not include any additional convolutional layer that is trained for a separate processing of a FC, and does not include any additional convolutional layer that is trained for a separate processing of a SC. It is to be understood, however, that some embodiments contemplate a set **41** which comprises more than one hidden convolutional layer **46** trained for separately processing FC **20**, some embodiments contemplate a set **41** which comprises more than one hidden convolutional layer **48** trained for separately processing SC **36.**

Outputs **50** and **52** are optionally and preferably combined to produce a combined output **54.** Typically, each activation map of output **50** is combined with an activation map of output **52.** For example, the respective activation maps of outputs **50** and **52** can be concatenated along one of their dimensions x, y, or z in which case combined output **54** is a three-dimensional concatenated output. When each layers **46** and **48** is associated with a single kernel, or when row kernels are employed as further detailed hereinabove, the respective outputs **50** and **52** are, as stated, two-dimensional outputs, and so combined output **54** is a two-dimensional concatenated output.

The concatenation is optionally and preferably along the depth dimension of the outputs **50** and **52.** Preferably, layers **46** and **48** have the same lateral dimensions so that concatenated output **54** has a depth that equals the sum of the depths of each of outputs **50** and **52.** For example, if the dimensions of layer **46** is (X×Y×N₄₆) and the dimensions of layer **48** is X×Y×N₄₈, where X is the number of neurons in a first lateral dimension, Y is the number of neurons in a second lateral dimension, N₄₆ is the number of kernels employed in layer **46,** and N₄₈ is the number of kernels employed in layer **48,** then in this embodiment, the dimensions of concatenated output **54** is X×Y×( N₄₆+N₄₈). In this case concatenated output **54** can be viewed as a cuboid.

Also contemplated are embodiments in which layers **46** and **48** do not have the same lateral dimensions. In these embodiments, concatenated output **54** does not have a fixed lateral dimensions along its depth. In this case concatenated output **54** can be viewed as two cuboids of different dimensions that are a stacked along their depth dimensions.

Further contemplated, are embodiments in which the respective activation maps of outputs **50** and **52** are concatenated along one of their lateral dimensions. In these embodiments, when the depths of outputs **50** and **52** are the same (namely, when each of layers **46** and **48** is associated with the same number of kernels), concatenated output **54** has a uniform depth that equals the depth of each of outputs **50** and **52,** and when layers **46** and **48** have different depth, concatenated output **54** can have a non-uniform depth, wherein those parts of output **54** that originate from output **50** have the depth of output **50,** and those parts of output **54** that originate from output **52** have the depth of output **52.** Alternatively, two or more activation maps of the same output can be combined before concatenation, so as to provide a concatenated output having a uniform depth.

CNN **40** also comprises an additional set of layers **56** trained for processing combined output **54.** Set **56** preferably comprises one or more hidden convolutional layers **58** that receive combined output **54.** Thus, unlike set **41** which applies the kernels separately to the FC and SC, and therefore provide activation maps that are exclusive to either the FC or the SC, the convolutional layers of set **56** apply the kernels conjointly both to activation values obtained from the FC and to activation values obtained from the SC. Such a conjoint convolution provides, for each kernel, an activation map that includes mixed activation values. The activation values are mixed in the sense that each mixed activation value is obtained both from activation values provided by layer **46** based on the FC, and from activation values provided by layer **48** based on the SC.

The number of hidden convolutional layers **58** of set **56** is optionally and preferably at least one, or at least two, or at least three, or at least four. For clarity of presentation, the output of the layers in set **56** is not shown.

The set **56** can optionally and preferably also comprises one or more fully connected layers **60.** Preferably, but not necessarily, fully connected layer **60** receives its input from the last convolutional layer **58** of set **56.** Fully connected layer **60** can be designed to receive a one-dimensional vector that is generated by a concatenation of the activation maps generated by the last convolutional layer **58.** Fully connected layer **60** typically employs a nonlinear activation function, such as, but not limited to, a sigmoid. Set **56** can also comprise an output layer **62** that stores output values indicative of a particular condition (or its absence) and/or prediction of outcome, and/or prediction of a response to a treatment and/or classification to treatment response group, of a subject whose brain is described by the input FC **20** and SC **36.** In these embodiments CNN **40** is trained for determining this particular condition (*e.g.*, neurological condition, psychiatric condition). Specifically, in these embodiments the training data used by the CNN training program includes both FCs and SCs that are labeled as characterizing subjects that have been diagnosed with the particular condition, and/or labeled with a particular outcome or classification to a specific treatment response group, and FCs and SCs that are labeled as characterizing control subjects.

Representative examples of particular conditions for which the CNN of the present embodiments can be trained, include, without limitation, psychiatric condition, traumatic brain injury (TBI), mild TBI (*e.g.,* brain concussion), depression disorder (e.g., major depression disorder, persistent depressive disorder), posttraumatic stress disorder (PTSD), pain (*e.g.,* acute pain, chronic pain, mechanical pain, static allodynia, dynamic allodynia, bone cancer pain, headache, osteoarthritic pain, inflammatory pain, and pain associated with autoimmune disorders or fibromyalgia), stroke, epilepsy, Parkinson, multiple sclerosis, agitation, Alzheimer's disease and/or dementia, anxiety disorder, panic disorder, phobic disorder, bipolar disorder, borderline personality disorder, behavior control problems, body dysmorphic disorder, cognitive problem (*e.g.,* mild cognitive impairment), dissociative disorders, eating disorder, appetite disorder, fatigue, impulse-control problems, irritability, mood problems, movement problems, obsessive-compulsive disorder, personality disorders, schizophrenia and other psychotic disorders, seasonal affective disorder, sexual disorders, sleep disorders, stuttering, Tourette's Syndrome, Trichotillomania, and violent/self-destructive behaviors.

Preferably, output layer **62** stores the likelihood that the subject has a neurological condition, and/or the likelihood that the subject has a psychiatric condition, and/or the likelihood that the subject has a particular outcome and/or a prediction of a response of the subject to a treatment and/or a classification of the subject to a specific treatment response group.

Output layer **62** can alternatively or additionally store output values indicative of a type of the neurological or psychiatric condition that the subject has. In these embodiments CNN **40** is trained for determining this particular neurological and/or psychiatric condition. Specifically, in these embodiments the training data used by the CNN training program include both FCs and SCs that are labeled as characterizing subjects that have been diagnosed with the particular neurological and/or psychiatric condition, and FCs and SCs that are labeled as characterizing control subjects. Preferably, output layer **62** stores the likelihood that the subject has the neurological and/or psychiatric condition.

CNN **40** can alternatively be trained for determining whether or not the subject has a non-specific condition (*e.g.*, neurological, psychiatric), and/or for determining the type of this condition. Specifically, in these embodiments the training data used by the CNN training program include FCs and SCs of a cohort of subjects, diagnosed with a diversity of conditions (*e.g.*, neurological conditions and/or psychiatric conditions), including control subjects. Preferably, output layer **62** stores the likelihood that the subject has one or more of the condition of the diversity.

Referring back to FIG. 1, the method continues to **15** at which the CNN (*e.g.,* CNN **40**) is fed with the subject-specific FC and SC (*e.g.,* FC **20** and SC **36**), and to **16** at which an output indicative of the condition, and/or a prediction of clinical outcome and/or a prediction of a response of the subject to a treatment and/or a classification of the subject to treatment response groups of a subject is received from the CNN. In some embodiments of the present invention the method proceeds to **17** at which a report pertaining to the condition and/or the clinical outcome and/or treatment response and/or to the likelihood is that the subject has the condition is generated. The report can be displayed on a display device or transmitted to a computer readable medium. The report can be presented as text, and/or graphically and/or using a color index.

The method optionally continues to **18** at which the subject is treated for the condition, responsively to the output from the CNN. For example, when the likelihood that the subject has a particular condition (*e.g.*, neurological condition, psychiatric condition) is above a predetermined threshold, the subject can be treated for that particular condition based on the CNN's output. The treatment can comprise a pharmacological treatment employing an active agent selected to treat particular condition, or a surgical intervention, and/or a rehabilitative treatment, and/or phototherapy, and/or hyperbaric therapy, and/or neurofeedback, and/or magnetic stimulation and/or electric stimulation.

In some embodiments of the present invention operation **18** is not part of the method.

The method ends at **19.**

The method of the present embodiments can be used in more than one way. In some embodiments of the present invention the method is used for determining whether or not a subject has a particular condition (*e.g.*, neurological condition, psychiatric condition), and optionally and preferably also the likelihood that the subject has that particular condition. In these embodiments, the method employs a CNN that is trained for that particular condition.

In some embodiments of the present invention the method is used for determining whether or not a subject has a non-specific condition (*e.g.*, neurological condition, psychiatric condition), and optionally and preferably also the likelihood that the subject has a non-specific neurological and/or psychiatric condition. In these embodiments, the method employs a CNN that is trained for a non-specific condition.

In some embodiments of the present invention the method is used for identifying the condition (*e.g.*, neurological condition, psychiatric condition) of the subject. In these embodiments, the method employs a CNN that is trained for a non-specific condition, but also trained to identify the condition. Alternatively, selected operations of the method can be reexecuted a plurality of times, each time using a CNN trained for a different particular condition. Once the output of one of the CNNs indicates a likelihood which is above a predetermined threshold, the method can determine that it is likely that the subject has the condition for which the respective CNN was trained.

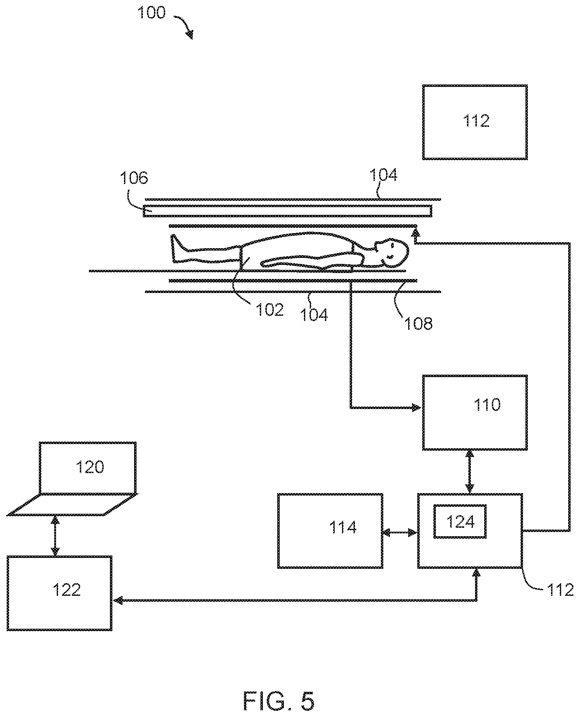

Reference is now made to FIG. 5 which is a schematic illustration of an MRI system **100** for imaging a body **102,** according to some embodiments of the present invention. System **100** comprises a static magnet system **104** which generates a substantially homogeneous and stationary magnetic field B₀ in the longitudinal direction, a gradient assembly **106** which generates instantaneous magnetic field gradient pulses to form a non-uniform superimposed magnetic field, and a radiofrequency transmitter system **108** which generates and transmits radiofrequency pulses to body **102.**

System **100** further comprises an acquisition system **110** which acquires MR signal from the body, and a control system **112** which is configured for implementing a pulse sequence, as further detailed hereinabove. In particular, control system **112** is configured for allowing the operator to select at least between a pulse sequence that is suitable for acquiring an MR signal from which fMRI data can be obtained, and a pulse sequence suitable for acquiring an MR signal from which structural and functional data can be obtained. Control system **112** is also configured to control acquisition system **110** such that MR signals are sequentially acquired.

In various exemplary embodiments of the invention system **100** further comprises an image producing system **114** which produces MR images from the signals. Image producing system **114** optionally and preferably implements a Fourier transform so as to transform the data into an array of image data.

The operation of system **100** is preferably controlled from an operator console **120** which can include a keyboard, control panel a display, and the like. Console **120** can include or it can communicate with a data processor **122.**

Gradient pulses and/or whole body pulses can be generated by a generator module **124** which is typically a part of control system **112.** Generator module **124** produces data which indicates the timing, strength and shape of the radiofrequency pulses which are to be produced, and the timing of and length of the data acquisition window.

Gradient assembly **106** typically comprises *Gₓ*, *G_{y}* and *G_{z}* coils each producing the magnetic field gradients used for position encoding acquired signals. Radiofrequency transmitter system **108** is typically a resonator which is used both for transmitting the radiofrequency signals and for sensing the resulting signals radiated by the excited nuclei in body **102.** The sensed MR signals can be demodulated, filtered, digitized *etc.* in acquisition system **110** or control system **112.**

Data processor **122** is preferably configured for receiving the signals from control system **112,** generate functional MR data and structural MR data, and execute selected operations of the method as further detailed hereinabove.

As used herein the term "about" refers to ± 10 %

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

The following Examples demonstrate diagnosis of brain disorders and prediction of clinical outcome or treatment response based on connectomes. Specifically, these Examples demonstrate classification of subjects with mild traumatic brain injury who are considered as suffering from concussion, and prediction of clinical outcome of subjects with concussion based on cognition assessed a year post-injury. The following Examples also demonstrate classification of subjects with major depressive disorder (MDD), and prediction of treatment response in subjects with MDD treated with selective serotonin reuptake inhibitors (SSRIs), 8 weeks after starting treatment.

### EXAMPLE 1

### Methods

### Experiment 1: Concussion

### Participants

A total of 102 subjects were enrolled in this study. Sixty-six subjects with diffuse closed head injury, including 38 mild (mTBI) and 28 moderate-severe (msTBI), were recruited from the Neurosurgery Department, Head Rehabilitation Department and the Emergency Room, Sheba Medical Center, Tel-Hashomer. Thirty-six age and gender matched healthy controls with no history of other neurological or psychiatric disorders were recruited from the community.

Injury severity was defined using the Glasgow Coma Scale (GCS) within the first 24 hours of hospital admission after injury, and was divided into two categories of mild, and moderate-severe injury. GCS scores of 14-15 were considered "mild", while GCS 3-13 were considered "moderate-severe". Mild TBI subjects were also categorized as complicated mTBI (cmTBI), if their anatomical MRI scan showed signs of injury such as contusions or diffuse axonal injury which were not observed at initial CT scan, and concussion if displayed no morphological injuries in both CT and MRI scans.

Mild TBI subjects were scanned within two weeks following their injury, moderate-severe subjects were scanned as soon as possible, within 4 months from time of injury, depending on their clinical status. Furthermore, subjects were followed for a year from injury and both cognitive and MRI scans were performed. The following inclusion criteria were used for TBI participants:
1) Male and female participants, aged 18-65 years old.
2) TBI subjects who had suffered a diffuse close head injury.
3) Subjects who are fully conscious, able to complete and sign hospitalization.

Exclusion criteria for both TBI and healthy control groups were:
1) History of other major neurological or psychiatric illnesses.
2) Use of medication that is likely to substantially affect cognitive performance.
3) Subjects with large lesions on MRI were excluded.
4) Exclusion criteria of MRI: claustrophobia and presence of metal in the body.

### Image Acquisition

Participants underwent a full MRI protocol on a 3 Tesla Philips Ingenia scanner using a 32-channel radio frequency coil, at the Division of Diagnostic Imaging, Sheba Medical Center. The MRI protocol included: resting-state fMRI, and a DWI scan. T₁-weighted and T₂ FLAIR scans were additionally performed for registration, clinical diagnosis and lesion evaluation.

High-resolution images of the entire brain were acquired for each subject using a turbo field echo (TFE) weighted sequence with the following parameters: TR=7.9s, TE=3.5ms, flip angle=8°, FOV of 256 mm, voxel size = 1 mm². Functional MRI images were acquired during rest with the following parameters: TR=2s, TE=32ms, flip angle=77°, matrix=80×80 and FOV of 240 mm², voxel size =3 mm² with a gap of 0.4mm, 300 volumes. Spin-echo diffusion weighted echo-planar imaging (DWI-EPI) sequence was performed with the following parameters: 70 axial slices with in-plane resolution of 2 mm² and slice thickness = 2 mm; repetition time (TR)=8500ms, echo time (TE)=92ms, matrix=96×96 and field of view (FOV) of 192 mm². 32 diffusion weighted images were acquired in isotropically distributed directions, with b=1000s²/mm (Δ/δ=33/26 ms) and an additional non-DWI image (b₀).

### Clinical Outcome: Cognitive Assessment

Twenty healthy controls, 14 concussed subjects and 13 moderate-severe subjects completed a cognitive assessment using the computerized neuropsychological assessment battery (CANTAB), a year post injury. The cognitive tests covered several broad domains, including executive functioning, memory, and sustained attention. The following tests were administered: Paired Associates Learning (PAL), Delayed Matching to Sample (DMS), Spatial Span (SSP), Spatial Working Memory (SWM), Reaction Time (RTI), Rapid Visual Information Processing (RVP), One Touch Stockings of Cambridge (OTS), Attention Switching Task (AST). The cognitive tests were administered according to standardized instructions by trained and research assistants and advanced undergraduate students. Test scores were entered into a factor analysis in order to create one overall cognitive factor.

### Experiment 2: Major Depression Disorder

### Participants

A total of 46 subjects were enrolled in this study. Twenty-three subjects with a diagnosis of major depressive disorder and 23 age and gender matched healthy controls. Subjects were recruited from the community and through the Psychiatric Department, Sheba Medical Center.

Inclusion criteria were:
1) Aged 18-65 year old.
2) Able to complete and sign an informed consent.

Additional criteria for subjects were:
1) Major depressive disorder diagnosis based on the MINI 7.0.2 questionnaire and DSM criteria.
2) Subjects who are in need for selective serotonin reuptake inhibitors (SSRIs) and have had no more than 2 prior attempts of antidepressant treatment.

Healthy controls were recruited from the community and were examined by a certified psychiatric to exclude depressive symptoms.

The Institutional Ethics Committee of the Sheba Medical Center, Tel Hashomer, approved both studies and all subjects signed an informed consent. The studies were carried out in accordance with the relevant guidelines and regulations of the ethics committee.

### Image Acquisition

Participants underwent a full MRI protocol, on a 3T MAGNETOM PRISMA Siemens scanner using a 64-channel radio frequency coil, at the Division of Diagnostic Imaging, Sheba Medical Center. The MRI protocol included: resting-state fMRI, and a DWI scan. A T₁-weighted scan was performed for registration purposes.

High-resolution images of the entire brain were acquired for each subject using a magnetization-prepared rapid acquisition gradient echo (MPRAGE) sequence with the following parameters: TR=2.1s, TE=2.45ms, flip angle=8°, FOV of 256 mm, voxel size =1 mm². Functional MRI images were acquired during rest using a multi-band accelerated echo-planar imaging (MB-EPI) sequence with the following parameters: TR=720ms, TE=33ms, flip angle=52°, matrix=104x90 and FOV of 208×180mm², voxel size=2 mm² with 800 volumes. A diffusion multi-band accelerated echo-planar imaging (MB-EPI) sequence was performed with the following parameters: 72 axial slices with in-plane resolution of 2 mm² and slice thickness = 2 mm; TR=3 s, TE=56.8 ms, matrix=86×112 and FOV of 224×172 mm². 64 diffusion weighted images were acquired in isotropically distributed directions, with b=1000s²/mm (Δ/δ=27/10 ms) and 4 additional non-DWI images (b₀).

### Clinical Outcome: Treatment Response

Subjects with MDD were administered at initial visit a Selective Serotonin Reuptake Inhibitor antidepressant medication and were followed for a period of 8 weeks. Subjects were administered the Hamilton Depression Rating Scale prior treatment and 8 weeks post treatment. Subjects were classified as "treatment responders" or "non-responders" based on 50% change and above, in the Hamilton questionnaire from the initial visit.

### Image Preprocessing

### Functional connectivity preprocessing

Functional image preprocessing was performed using CONN toolbox v.19.c. (www(dot)nitrc(dot)org/projects/conn, RRID:SCR_009550). The preprocessing consisted of the following steps. Spatial preprocessing comprising of (1) realignment of the functional images to a mean functional image by rigid body transformation (translations and rotations) to correct for head motions and unwarping the images to remove additional motion artifacts. (2) direct co-registration of individual images to functional images. (3) tissue segmentation of grey-matter (GM), white-matter (WM) and cerebro-spinal fluid (CSF) tissue maps. Thereafter temporal preprocessing was executed in order to increase the robustness of the data. CompCor method, built in the CONN toolbox, was used to determine noise ROIs (non-neuronal noise; WM, CSF as well as noise from other sources) by regressing out principal components from noise ROIs. Significant principal components were then introduced as covariates in the general linear model as an estimate of the non-neuronal noise signal space. These covariates were removed from the functional data using linear regression, and the resulting residual fMRI blood-oxygenation-dependent (BOLD) time series were band-pass filtered (0.008< ∞ Hz) in order to obtain a highfrequency range of interest.

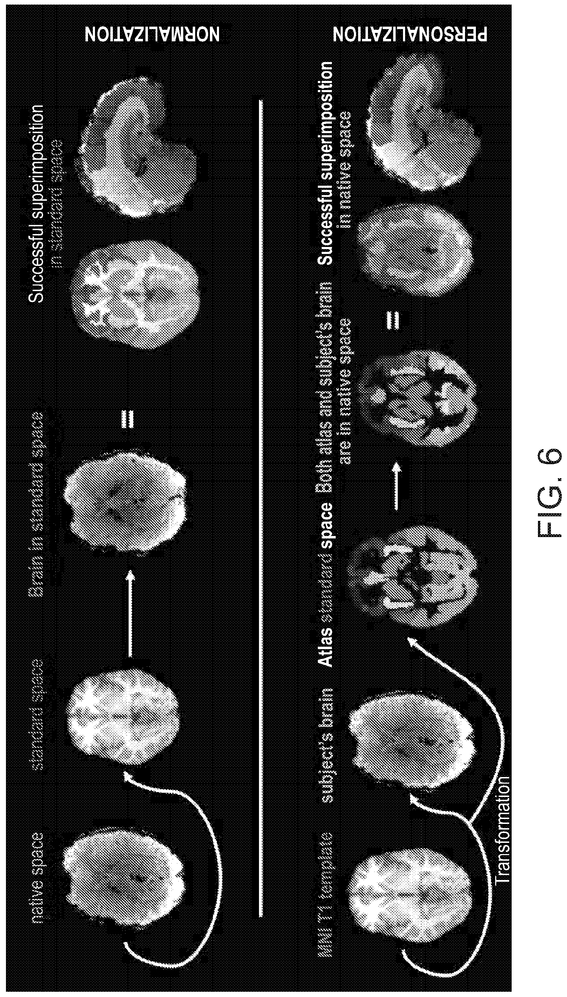

To establish a personalized connectome fingerprint that would be more representative of each individual's network, the Inventors remained in the subject's native space while still allowing parcellation by atlas images. More specifically, rather than normalizing the images to the standard space (FIG. 6, top panel), the images were personalized by creating subject-specific template and atlas label images (FIG. 6, bottom panel). This process was proceeded as follows. The Brainnetome atlas was used for obtaining 273 cortical, subcortical and cerebellar regions. A skull stripped resting-state averaged image was created by averaging over all the resting-state scan volumes. This resting-state averaged image is representative of the subject's native space. Then, a structural MNI template image or a parceled and segmented T1 image in the standard space was warped to fit to the space of the averaged resting-state image in a process called registration. This registration was a linear transformation of the image, that includes translations, rotations, rescale and shear in all orthogonal Cartesian X-Y-Z axes. This linear transformation was conducted using FLIRT in FSL (FMRIB's Linear Image Registration Tool) for each subject.

This registration process resulted in subject-specific template images that can be superimposed on the native space resting-state images of each subject. Following a successful registration, the transformation matrix which encompassed all the linear transformations that were needed to register the MNI image from the standard space to the subject's native space was recorded. This transformation matrix was created for each subject and was then used to apply the same linear transformation to the Brainnetome atlas. This registered the Brainnetome atlas to each subject's individual space, thus creating a personalized, subject-specific Brainnetome atlas image per each subject, which could further be precisely superimposed on each subject's native space resting-state images.

In order to create the correlation matrices from the denoised functional images, in-house MATLAB scripts performed the following analysis steps per subject: (1) The BOLD signal was averaged across all voxels of each brain area, for each time point, thus creating a BOLD intensity time-series of each brain area. (2) Pearson correlations were calculated between BOLD intensity time-series of each pair of brain areas, resulting in symmetrical correlation matrices containing 273 rows and columns corresponding to all the Brainnetome's areas as nodes and correlations as edges.

### Structural connectivity preprocessing

A DTI-based network was constructed using ExploreDTI v4.8.6, and included the following processing pipeline: (1) regularization: images were regularized and resampled (regularization factor of 0.5). (2) Brain extraction: automatic skull stripping and additional manual cleaning to remove areas of remaining skull and eyes. (3) Motion correction: diffusion data was corrected for motion and eddy currents distortions using rigid-body transformations to further minimize motion artifacts. (4) DTI calculation: the diffusion tensors were calculated using a non-linear regression procedure.

Subject-specific template and atlas label images were created for each subject in the subject's native space. First, a skull stripped averaged image of all the DWI volumes was created per each subject. Then, a structural MNI template image was registered to the skull stripped averaged DWI image. The registration transformation matrix was then also implemented on the Brainnetome atlas, thus creating both a template and an atlas image in the native space of each subject. These Images were then used to define the edges between each pair of nodes.

Whole brain tractography was performed based on a deterministic streamline fiber tractography approach. Trajectory propagation was terminated if the angle between consecutive steps exceeded 45° or if the fractional anisotropy (FA) values were lower than 0.2. The step size was set to 1 mm. Edges were defined by the number of streamlines connecting each pair of nodes. Since DTI tractography does not differentiate between efferent and afferent fibers, the reconstructed graphs were all undirected. The resulting number of streamlines was converted to square, symmetrical connectivity matrices with 273 rows and columns, corresponding to the Brainnetome brain areas. Connections with fewer than 10 streamlines were treated as noise and were given a value of zero.

### Classification and Prediction Framework

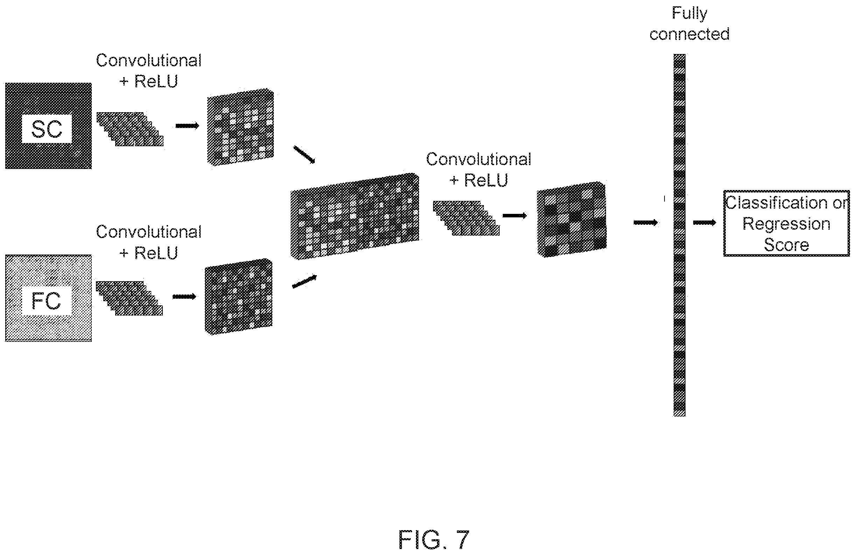

A CNN which integrates two types of connectomes in a middle fusion approach, Middle Fusion Convolutional Neural Network (MFCNN) was constructed. The CNN architecture employed in this Example is illustrated in FIG. 7. The CNN architecture's input layer consisted of two connectome features (FC and SC), followed by five hidden layers, and an output layer consisting of values indicating either the classification or the prediction scores. Within the five hidden layers, one layer applied a 2D convolution on both modalities separately followed by a ReLU function to preserve nonlinearity. Since a 2D convolution was applied on 2D data, the two outputs are in the form of a cuboid (the third axis represents the number of kernels of the respective first layer), one for each modality. These two cuboids were then concatenated along one of the lateral dimensions, to one fused cuboid containing activation values from both modalities. This fused cuboid was then propagated through an additional 2D convolutional layer and then by a fully connected layer, while each of the layers were followed by a ReLU. A final fully connected layer was followed by a softmax activation function (in the case of classification) or by no activation function (in the case of regression prediction).

In case of disease classification or treatment response classification, the final output layer consisted of two numbers each representing the likelihood of the subject to belong to one of two classes. In case of prediction of clinical outcome, the final output layer consisted of one number representing prediction of cognitive score.

Weight update during training was done by the Adam optimizer [arXiv:1412.6980], for classification, and Stochastic Gradient Descent, for regression prediction. Regularization was applied by using dropout in each layer, before and after the concatenation.

The CNN was implemented in Python using TensorFlow, Keras packages, and Pytorch.

Both datasets were divided into training, validation, and test sets for the different classification and prediction purposes. For classification purposes, the dataset was split to 70%-80% for the training and validation, and 20%-30% for the test set. In all cases the validation set was kept as small as possible to save as much data for training and testing as possible.

Additional consideration in data splitting for each of the two experiments were: (1) Eight mTBI subjects were considered complicated (see above), and therefore were included only in the training set. (2) For the prediction of cognitive outcome a year post-injury several moderate-severe TBI subjects were added to the training set, due to missing data in some of the concussed subjects, and in order to increase the variance of cognitive deficits. (3) For the prediction of treatment response in the MDD subjects the dataset was significantly smaller, and therefore a 9-fold cross validation method was employed. Further details regarding data splitting are summarized in Table 1, below.

Classification ability was assessed by various scores: accuracy, area under curve (AUC), sensitivity, specificity, positive predictive value (PPV) and negative predictive value (NPV). Prediction of clinical outcome was assessed using Pearson correlation coefficients and p-value. Table 1, below, summarized the training, validation, and testing datasets used in this Example.

**Table 1**

| Disease Classification | | | |
|---|---|---|---|
| **Concussion Classification** | **Train** | **Validation** | **Test** |
| Healthy Controls | 16 | 2 | 18 |
| Concussion | 10 | 2 | 18 |
| Complicated mTBI | 8 | 0 | 0 |

| **MDD Classification** | **Train** | **Validation** | **Test** |
|---|---|---|---|
| Healthy Controls | 15 | 3 | 5 |
| MDD | 15 | 3 | 5 |

| Prediction of Clinical Outcome | | | |
|---|---|---|---|
| **Cognitive Outcome** | **Train** | **Validation** | **Test** |
| Healthy Controls | 16 | 4 | 0 |
| mTBI | 0 | 3 | 11 |
| Moderate-severe TBI | 13 | 0 | 0 |

| **Treatment Response** | **Train** | **Validation** | **Test** |
|---|---|---|---|
| MDD- responders | 7 | 1 | 1 |
| MDD- non-responders | 7 | 1 | 1 |

### Additional Experiments

To examine the contribution of several neuroimaging analyses components, several additional sets of experiments were conducted.

A first set of additional experiments was conducted to examine the effect of using both functional and structural connectomes compared to that of using only one of the modalities. This set of experiments compared between performances when using SC+FC (both structural and functional connectomes), FC+FC (using each participant's functional connectome twice, inputted into the same CNN architecture shown in FIG. 7), and SC+SC (using each participant's structural connectome twice, inputted into the same CNN architecture shown in FIG. 7).

A second set of additional experiments was conducted to examine the effect of using functional personalized connectomes (P) compared to that of functional normalized connectomes (N).

A third set of additional experiments was conducted to examine the effect of using a Brainnetome atlas parcellation of 273 ROI's compared to that of using the automated anatomical labeling atlas (AAL) atlas parcellation of 116 ROI's.

A fourth set of additional experiments was conducted to examine the effect of using a square kernel compared to a row kernel. Row kernel length was equal to the length of the connectome based on the number of ROI in the atlas. In this case the kernel was applied by the stride of 1 by moving each time one row down [arXiv:1707.06682]. Square kernel size was either defined as 3×3 or 2×2.

A fifth set of additional experiments was conducted to examine the effect of applying the concatenation of the structural and functional features at a different layer within in the CNN architecture: (a) fusion in the first layer (Early Fusion CNN), (b) fusion in the second layer (Middle Fusion CNN), and (c) fusion in the last layer (Late Fusion CNN).

### Results

The results for all the experiments are given in detail in Table 2, below. Across all analyses of disease classification, as well as in prediction of clinical outcome in both concussion and MDD, the middle fusion architecture (fusion in the second layer) using the row kernel and both modalities of structural and functional connectomes in the personalized connectome fingerprint format yielded the highest results.

**Table 2**

| Disease Classification | | | | | | |
|---|---|---|---|---|---|---|
| Concussion Classification | | | | | | |
| Row kernel | ACC | AUC | Sensitivity | Specificity | PPV | NPV |
| Early Fusion SC+FC (P) | 69.4% | 0.6944 | 94% | 44% | 63% | 89% |
| Middle Fusion SC+FC (P) | 91.4% | 0.9166 | 94% | 89% | 89% | 94% |
| Middle Fusion SC+SC (P) | 72.2% | 0.7222 | 56% | 89% | 83% | 67% |
| Middle Fusion SC+FC (N) | 69.4% | 0.6944 | 89% | 50% | 64% | 82% |

| Square Kernel | ACC | AUC | Sensitivity | Specificity | PPV | NPV |
|---|---|---|---|---|---|---|
| Early Fusion SC+FC (P) | 69.4% | 0.6944 | 100% | 39% | 62% | 100% |
| Middle Fusion SC+FC (P) | 80.6% | 0.8055 | 83% | 78% | 79% | 82% |
| Middle Fusion SC+SC (P) | 75% | 0.75 | 94% | 56% | 68% | 91% |
| Middle Fusion SC+FC (N) | 75% | 0.75 | 78% | 72% | 74% | 76% |

| MDD Classification | | | | | | |
|---|---|---|---|---|---|---|
| Row kernel | ACC | AUC | Sensitivity | Specificity | PPV | NPV |
| Early Fusion SC+FC (P) | 80.00% | 0.8 | 80.00% | 80.00% | 80.00% | 80.00% |
| Middle Fusion SC+FC (P) | 90.00% | 0.9 | 100.00% | 80.00% | 83.00% | 100.00% |
| Late Fusion SC+FC (P) | 50.00% | 0.5 | 40.00% | 60.00% | 50.00% | 50.00% |
| Middle Fusion FC+FC (P) | 60.00% | 0.6 | 80.00% | 40.00% | 57.00% | 67.00% |

| Square Kernel | ACC | AUC | Sensitivity | Specificity | PPV | NPV |
|---|---|---|---|---|---|---|
| Early Fusion SC+FC (P) | 60.00% | 0.6 | 40.00% | 80.00% | 67.00% | 57.00% |
| Middle Fusion SC+FC (P) | 60.00% | 0.6 | 20.00% | 100.00% | 100.00% | 56.00% |
| Late Fusion SC+FC (P) | 70.00% | 0.7 | 100.00% | 40.00% | 62.00% | 100.00% |
| Middle Fusion FC+FC (P) | 70.00% | 0.7 | 40.00% | 100.00% | 100.00% | 100.00% |
| Middle Fusion FC+FC (N) | 70.00% | 0.7 | 80.00% | 60.00% | 67.00% | 75.00% |

| Prediction of Clinical Outcome | | | | | | |
|---|---|---|---|---|---|---|
| Cognitive Outcome | | | | | | |
| Row kernel | *r* | *p-value* | | | | |
| Middle Fusion SC+FC (P) | 0.7101 | 0.0143 | | | | |

| Treatment Response | | | | | | |
|---|---|---|---|---|---|---|
| Row kernel | ACC | AUC | Sensitivity | Specificity | PPV | NPV |
| Middle Fusion | 83% | 0.83 | 88.89% | 77.78% | 80% | 87.5% |
| SC+FC (P) | | | | | | |

### Hyperparameters

For each comparative experiment different hyperparameters were used to achieve the highest validation accuracy, as summarized in Table 3, below. Hyperparameters of experiments that did not achieve more than 60% accuracy in the validation set were considered as non-significant and were excluded.

**Table 3**

| Disease Classification | | | | | | | | |
|---|---|---|---|---|---|---|---|---|
| Concussion Classification | | | | | | | | |
| | Dropout | 1LF | 1LF-FC | 1LF-SC | Dropout-FC | Dropout-SC | 2LF | 3LF |
| Early Fusion SC+FC (P) | 75.00% | 14 | | | | | 28 | 112 |
| Middle Fusion SC+FC (P) | 70% | | 16 | 12 | | | 56 | 112 |
| Middle Fusion SC+SC (P) | 60% | | 8 | 6 | | | 28 | 56 |
| Middle Fusion SC+FC (N) | 70% | | 16 | 12 | | | 56 | 112 |

| MDD Classification | | | | | | | | |
|---|---|---|---|---|---|---|---|---|
| Early Fusion SC+FC (P) | 85.00% | 14 | | | | | 140 | 560 |
| Middle Fusion SC+FC (P) | 85.00% | | 8 | 6 | | | 140 | 560 |
| Late Fusion SC+FC (P) | 95.00% | | 8 | 8 | 95% | 20% | 28 | 112 |
| Middle Fusion FC+FC (P) | 75.00% | | 8 | 6 | | | 1400 | 5600 |

| Prediction Of Clinical Outcome | | | | | | | | |
|---|---|---|---|---|---|---|---|---|
| Cognitive Outcome | | | | | | | | |
| Middle Fusion SC+FC (P) | 70.00% | | 8 | 6 | | | 2800 | 11200 |
| Treatment Response | | | | | | | | |

| Middle Fusion SC+FC (P) | | | | | | | | |
|---|---|---|---|---|---|---|---|---|
| Split 1 | 95% | | 8 | 6 | | | 3360 | 13440 |
| Split 2 | 90% | | 8 | 6 | | | 1400 | 5600 |
| Split 3 | 70% | | 8 | 6 | | | 1400 | 5600 |
| Split 4 | 95% | | 8 | 6 | | | 2240 | 8960 |
| Split 5 | 95% | | 8 | 6 | | | 2240 | 8960 |
| Split 6 | 65% | | 8 | 6 | | | 1680 | 6720 |
| Split 7 | 95% | | 8 | 6 | | | 1400 | 5600 |
| Split 8 | 95% | | 8 | 6 | | | 2240 | 8960 |
| Split 9 | 85% | | 8 | 6 | | | 2240 | 8960 |

| | | | | | | | | |
|---|---|---|---|---|---|---|---|---|
| 1LF/ 2LF/ 3LF - 1st, 2nd and 3rd layer number of filters/features. Kernel size was 273×1. | | | | | | | | |

### EXAMPLE 2

Additional MDD patients and healthy controls subjects were recruited for the study. A total of 96 subjects were analyzed: Forty-seven subjects with a diagnosis of major depressive disorder and 49 age and gender matched healthy controls. SC and FC connectomes were created as detailed in Example 1.

The preprocessing of the functional connectivity and structural connectivity were as described in Example 1, except that in this Example, the Desikan-Killiany atlas was used instead of the Brainnetome atlas.

The architecture of the CNN that was employed in this Example is similar to the architecture described in Example 1, except that a single fully connected layer was employed (rather than the two applied in Example 1). The fully connected was followed by a softmax activation function in the case of classification, or by no activation function in the case of regression prediction.

Table 4, below, summarized the training, validation, and testing datasets used in this Example.

**Table 4**

| **MDD Classification** | **Train** | **Validation** | **Test** |
|---|---|---|---|
| Healthy Controls | 39 | 0 | 10 |
| MDD | 37 | 0 | 10 |

| Prediction of Clinical Outcome | | | |
|---|---|---|---|
| **Cognitive Outcome** | **Train** | **Validation** | **Test** |
| Healthy Controls | 16 | 4 | 0 |
| mTBI | 0 | 3 | 11 |
| Moderate-severe TBI | 13 | 0 | 0 |

| **Treatment Response** | **Train** | **Validation** | **Test** |
|---|---|---|---|
| MDD- responders | 8 | 0 | 5 |
| MDD- non-responders | 8 | 0 | 5 |

### Results

The results for the MDD experiment with the additional participants are given in detail in Table 5, below. Since across all initial analyses of disease classification, as well as prediction of clinical outcome in both concussion and MDD, the middle fusion architecture (fusion in the second layer) using the row kernel and both modalities of structural and functional connectomes in the personalized connectome fingerprint format yielded the highest results, only the following set of analysis were conducted on the enlarged MDD cohort.

**Table 5**

| Disease Classification | | | | | | |
|---|---|---|---|---|---|---|
| MDD Classification | | | | | | |
| | ACC | AUC | Sensitivity | Specificity | PPV | NPV |
| Early Fusion SC+FC (P) | 65% | 0.65 | 90% | 40% | 60% | 80% |
| **Middle Fusion SC+FC (P)** | **90%** | **0.9** | **100%** | **80%** | **100%** | **83.3%** |
| Late Fusion SC+FC (P) | 85% | 0.85 | 90% | 80% | 88.8% | 81.8% |
| Middle Fusion FC+FC (P) | 85% | 0.85 | 70% | 100% | 76.9% | 100% |
| Middle Fusion SC+SC (P) | 65% | 0.65 | 50% | 80% | 61.5% | 71.4% |
| Middle Fusion SC+FC (N) | 70% | 0.7 | 70% | 70% | 70% | 70% |

| **Treatment Response** | | | | | | |
|---|---|---|---|---|---|---|
| | ACC | AUC | Sensitivity | Specificity | PPV | NPV |
| Middle Fusion SC+FC (P) | 90% | 0.9 | 100% | 80% | 100% | 83.3% |

### Hyperparameters

For each comparative experiment different hyperparameters were used to achieve the highest validation accuracy, as summarized in Table 6, below. Hyperparameters of experiments that did not achieve more than 60% accuracy in the validation set were considered as non-significant and were excluded.

**Table 6**

| Disease Classification | | | | | | | | |
|---|---|---|---|---|---|---|---|---|
| MDD Classification | | | | | | | | |
| Row kernel | Dropout | 1LF | 1LF-FC | 1LF-SC | 2LF-FC | 2LF-SC | 2LF | 3LF |
| Early Fusion SC+FC (P) | 87% | 15 | | | | | 5 | 2 |
| **Middle Fusion SC+FC (P)** | 92% | | 50 | 50 | | | 32 | 2 |
| Late Fusion SC+FC (P) | 94% | | 200 | 200 | 64 | 64 | | 2 |
| Middle Fusion FC+FC (P) | 88% | | 32 | 32 | | | 16 | 2 |
| Middle Fusion SC+SC (P) | 83% | | 10 | 10 | | | 16 | 2 |
| Middle Fusion SC+FC (N) | 87% | | 15 | 15 | | | 5 | 2 |

| **Treatment Response** | | | | | | | | |
|---|---|---|---|---|---|---|---|---|
| Middle Fusion SC+FC (P) | 87% | | 60 | 60 | | | 15 | 2 |

| | | | | | | | | |
|---|---|---|---|---|---|---|---|---|
| 1LF/ 2LF/ 3LF - 1st, 2nd and 3rd layer number of filters/features. Kernel size was 273×1. | | | | | | | | |

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims. In addition, citation or identification of any reference in this application shall not be construed as an admission that such reference is available as prior art to the present invention. To the extent that section headings are used, they should not be construed as necessarily limiting.

## Claims

1. A method (10) of determining a condition of a subject, the method comprising:
receiving (11), from a computer readable medium or a cloud storage facility, functional magnetic resonance (MR) data and structural MR data, each describing the brain of the subject in a respective native space of said brain;
applying (12) a transformation of images of an anatomical atlas defined over a standardized space onto said respective native space, to provide respective parcellated functional MR data and structural MR data over said respective native space;
separately constructing (13): (i) a subject-specific functional connectome (FC) (20) using said functional MR data, and (ii) a subject-specific structural connectome (SC) (36) using said structural MR data;
accessing (14) a computer readable medium storing a trained convolutional neural network (CNN) (40) having a first set of layers (41) trained for separately processing FC (20) and SC (36), and a second set of layers (56) trained for processing combined outputs (54) from said first set of layers (41);
feeding (15) said CNN (40) with said subject-specific FC (20) and SC (36); and
receiving (16) from said second set of layers (56) an output indicative of a condition of the subject;
wherein said constructing (13) said subject-specific FC, comprises extracting from said functional MR data a plurality of time-ordered series of values, each series corresponding to a different region of said brain, and constructing a correlation matrix (20) describing correlation among said plurality of series, wherein said subject-specific FC (20) is said correlation matrix (20).

2. The method (10) according to claim 1, comprises calculating said transformation by receiving a mean MR image of said brain, and registering a template image defined over said standardized space onto said mean magnetic resonance image.

3. The method (10) according to claim 2, wherein said mean MR image is based on a volume average of at least one of said structural and functional MR data.

4. The method (10) according to any of claims 1-3, wherein said separate processing comprise generating a plurality of FC activation maps and a plurality of SC activation maps, and wherein said combined outputs comprise a concatenation between a respective FC activation map and respective SC activation map.

5. The method (10) according to any of claims 1-3, wherein said first set of layers (41) comprises one hidden convolutional layer (46) trained for separately processing FC (20), and one hidden convolutional layer (48) trained for separately processing SC (36).

6. The method (10) according to any of claims 1-3, wherein said first set of layers (41) comprises more than one hidden convolutional layer (46) trained for separately processing FC (20).

7. The method (10) according to any of claims 1-3, wherein said first set of layers (41) comprises more than one hidden convolutional layer (48) trained for separately processing SC (36).

8. The method (10) according to any of claims 1-6, wherein said second set of layers (56) comprises at least two hidden convolutional layers (58).

9. The method (10) according to any of claims 1-8, wherein said correlation is selected from the group consisting of a pairwise correlation, a partial correlation, and a distance correlation.

10. The method (10) according to any of claims 1-9, wherein said constructing said subject-specific SC (36), comprises applying whole brain tractography to define a plurality of streamlines or fractional anisotropy values between pairs of regions of said brain, and converting said plurality of streamlines or fractional anisotropy values to a connectivity matrix, wherein said subject-specific SC (36) is said connectivity matrix.

11. The method (10) according to any of claims 1-10, wherein said CNN (40)_is further trained for predicting a response to a treatment for the condition and/or a clinical outcome of the condition.

12. The method (10) according to any of claims 1-11, wherein said CNN (40) is trained for predicting a likelihood for at least one of: brain concussion, depressive disorder, stroke, traumatic brain injury, post-traumatic stress disorder, epilepsy, Parkinson, multiple sclerosis, agitation, abuse, Alzheimer's disease, anxiety, panic, phobic disorder, bipolar disorder, borderline personality disorder, behavior control disorder, body dysmorphic disorder, cognitive impairment, dissociative disorder, eating disorder, fatigue, impulse-control disorder, irritability, obsessive-compulsive disorder, personality disorders, psychotic disorder, sexual disorders, sleep disorder, stuttering, Tourette's Syndrome, Trichotillomania, self-destructive behavior, fibromyalgia, tremor, schizophrenia, attention-deficit disorder, hyperactivity disorder, and learning disorder.

13. A computer software product, comprising a computer-readable medium in which program instructions are stored, which instructions, when read by a data processor (122), cause the data processor (122) to receive functional MR data and structural MR data, each describing the brain of a subject, and execute the method (10) according to any of claims 1-12.

14. A magnetic resonance imaging (MRI) system (100) for imaging a brain of a subject (102), the system comprising:
an MRI scanner (104, 106,, 108) configured for scanning the brain to provide functional MR data and structural MR data, each describing the brain; and
a data processor (122) configured for executing the method according to any of claims 1-12.

## Patentansprüche

1. Verfahren (10) zur Bestimmung eines Zustands eines Probanden, wobei das Verfahren umfasst:
Empfangen (11), von einem computerlesbaren Medium oder einer Cloud-Speichereinrichtung, von funktionellen Magnetresonanz- (MR) Daten und strukturellen MR-Daten, die jeweils das Gehirns des Probanden in einem jeweiligen nativen Raum des Gehirns beschreiben;
Anwenden (12) einer Transformation von Bildern eines anatomischen Atlas, der über einen standardisierten Raum definiert ist, auf den jeweiligen nativen Raum, um jeweilige parzellierte funktionelle MR-Daten und strukturelle MR-Daten über den jeweiligen nativen Raum bereitzustellen;
getrenntes Erstellen (13): (i) eines probandenspezifischen funktionellen Konnektoms (FC) (20) unter Verwendung der funktionellen MR-Daten und (ii) eines probandenspezifischen strukturellen Konnektoms (SC) (36) unter Verwendung der strukturellen MR-Daten;
Zugreifen (14) auf ein computerlesbares Medium, das ein trainiertes konvolutionelles neuronales Netzwerk (CNN) (40) speichert, das einen ersten Satz von Schichten (41) aufweist, die für die getrennte Verarbeitung von FC (20) und SC (36) trainiert sind, sowie einen zweiten Satz von Schichten (56), die für die Verarbeitung kombinierter Ausgaben (54) aus dem ersten Satz von Schichten (41) trainiert sind;
Einspeisen (15) des CNN (40) mit dem probandenspezifischen FC (20) und SC (36); und
Empfangen (16) einer Ausgabe aus dem zweiten Satz von Schichten (56), die einen Zustand des Probanden anzeigt;
wobei das Erstellen (13) des probandenspezifischen FC das Extrahieren einer Vielzahl von zeitlich geordneten Wertreihen aus den funktionellen MR-Daten umfasst, wobei jede Reihe einer anderen Region des Gehirns entspricht, und das Erstellen einer Korrelationsmatrix (20), die die Korrelation zwischen der Vielzahl von Reihen beschreibt, wobei der probandenspezifische FC (20) die Korrelationsmatrix (20) ist.

2. Verfahren (10) nach Anspruch 1, das die Berechnung der Transformation durch Empfangen eines mittleren MR-Bildes des Gehirns und das Registrieren eines über den standardisierten Raum definierten Vorlagenbildes auf das mittlere Magnetresonanzbild umfasst.

3. Verfahren (10) nach Anspruch 2, wobei das mittlere MR-Bild basierend auf einem Volumendurchschnitt mindestens eines der strukturellen und funktionellen MR-Daten erstellt wird.

4. Verfahren (10) nach Anspruch 1-3, wobei die getrennte Verarbeitung das Erzeugen einer Vielzahl von FC-Aktivierungskarten und einer Vielzahl von SC-Aktivierungskarten umfasst, und wobei die kombinierten Ausgabedaten eine Verkettung zwischen einer jeweiligen FC-Aktivierungskarte und einer jeweiligen SC-Aktivierungskarte umfassen.

5. Verfahren (10) nach Anspruch 1-3, wobei der erste Satz von Schichten (41) eine versteckte Faltungsschicht (46), die für die separate Verarbeitung von FC (20) trainiert ist, und eine versteckte Faltungsschicht (48), die für die separate Verarbeitung von SC (36) trainiert ist, umfasst.

6. Verfahren (10) nach Anspruch 1-3, wobei der erste Satz von Schichten (41) mehr als eine verborgene Faltungsschicht (46) umfasst, die für die separate Verarbeitung von FC (20) trainiert wurde.

7. Verfahren (10) nach Anspruch 1-3, wobei der erste Satz von Schichten (41) mehr als eine verborgene Faltungsschicht (48) umfasst, die für die separate Verarbeitung von SC (36) trainiert wurde.

8. Verfahren (10) nach Anspruch 1-6, wobei der zweite Satz von Schichten (56) mindestens zwei verborgene Faltungsschichten (58) umfasst.

9. Verfahren (10) nach Anspruch 1-8, wobei die Korrelation aus der Gruppe ausgewählt ist, die aus einer paarweisen Korrelation, einer partiellen Korrelation und einer Abstandskorrelation besteht.

10. Verfahren (10) nach Anspruch 1-9, wobei das Erstellen der probandenspezifischen SC (36) umfasst: Anwenden einer Ganzhirn-Traktografie umfasst, um eine Vielzahl von Stromlinien oder fraktionalen Anisotropiewerte zwischen Paaren von Hirnregionen zu definieren, und Umwandeln der Vielzahl von Stromlinien oder fraktionalen Anisotropiewerte in eine Konnektivitätsmatrix, wobei die probandenspezifische SC (36) die Konnektivitätsmatrix ist.

11. Verfahren (10) nach Anspruch 1-10, wobei das CNN (40) ferner darauf trainiert ist, eine Reaktion auf eine Behandlung der Erkrankung und/oder einen klinischen Verlauf der Erkrankung vorherzusagen.

12. Verfahren (10) nach Anspruch 1-11, wobei das CNN (40) darauf trainiert ist, eine Wahrscheinlichkeit für mindestens eines der Folgenden vorherzusagen: eine Gehirnerschütterung, eine depressive Störung, einen Schlaganfall, eine traumatische Hirnverletzung, eine posttraumatische Belastungsstörung, eine Epilepsie, Parkinson-Krankheit, Multiple Sklerose, Unruhe, Misshandlung, Alzheimer-Krankheit, Angstzustände, Panik, eine phobische Störung, eine bipolare Störung, eine Borderline-Persönlichkeitsstörung, eine Verhaltenssteuerungsstörung, eine körperdysmorphe Störung, eine kognitive Beeinträchtigung, eine dissoziative Störung, eine Essstörung, Müdigkeit, eine Impulskontrollstörung, Reizbarkeit, eine Zwangsstörung, Persönlichkeitsstörungen, psychotische Störung, sexuelle Störungen, Schlafstörung, Stottern, Tourette-Syndrom, Trichotillomanie, selbstzerstörerisches Verhalten, Fibromyalgie, Tremor, Schizophrenie, Aufmerksamkeitsdefizitstörung, Hyperaktivitätsstörung und Lernstörung.

13. Computersoftwareprodukt, das ein computerlesbares Medium umfasst, auf dem Programmbefehle gespeichert sind, wobei diese Befehle, wenn sie von einem Datenprozessor (122) gelesen werden, bewirken, dass der Datenprozessor (122) funktionelle MR-Daten und strukturelle MR-Daten empfängt, die jeweils das Gehirn eines Probanden beschreiben, und das Verfahren (10) nach Anspruch 1-12 ausführt.

14. Magnetresonanzbildgebungs- (MRI) System (100) zur Bildgebung des Gehirns eines Probanden (102), wobei das System umfasst:
einen MRT-Scanner (104, 106, 108), der so konfiguriert ist, dass er das Gehirn abtastet, um funktionelle MR-Daten und strukturelle MR-Daten bereitzustellen, die jeweils das Gehirn beschreiben; und
einen Datenprozessor (122), der so konfiguriert ist, dass er das Verfahren nach Anspruch 1 bis 12 ausführt.

## Revendications

1. Procédé (10) permettant de déterminer l'état d'un sujet, le procédé comprenant :
la réception (11), en provenance d'un support lisible par ordinateur ou d'une infrastructure de stockage dans le cloud, de données de résonance magnétique (RM) fonctionnelle et de données de RM structurelle, chacune décrivant le cerveau du sujet dans un espace natif respectif dudit cerveau,
l'application (12), sur ledit espace natif respectif, d'une transformation des images d'un atlas anatomique défini dans un espace normalisé, afin de fournir des données de RM fonctionnelle et des données de RM structurelle respectives, parcellisées sur ledit espace natif respectif,
la construction (13) séparée : (i) d'un connectome fonctionnel (CF) (20) spécifique au sujet au moyen desdites données de RM fonctionnelle, et (ii) d'un connectome structurel (CS) (36) spécifique au sujet au moyen desdites données de RM structurelle,
l'accès (14) à un support lisible par ordinateur sur lequel est stocké un réseau neuronal convolutif (CNN, convolutional neural network) (40) entraîné présentant un premier ensemble de couches (41) entraîné à traiter séparément le CF (20) et le CS (36), et un deuxième ensemble de couches (56) entraîné à traiter des sorties combinées (54) provenant dudit premier ensemble de couches (41),
l'introduction (15) desdits CF (20) et CS (36) spécifiques au sujet dans ledit CNN (40), et
la réception (16), en provenance dudit deuxième ensemble de couches (56), d'une sortie indicative d'un état du sujet ;
ladite construction (13) dudit CF spécifique au sujet comprenant l'extraction, à partir desdites données de RM fonctionnelle, d'une pluralité de séries de valeurs dans l'ordre chronologique, chaque série correspondant à une région différente dudit cerveau, et la construction d'une matrice de corrélation (20) décrivant une corrélation entre ladite pluralité de séries, ledit CF spécifique au sujet (20) étant ladite matrice de corrélation (20).

2. Procédé (10) selon la revendication 1, comprenant le calcul de ladite transformation par réception d'une image RM moyenne dudit cerveau, et par recalage d'une image modèle définie dans ledit espace normalisé sur ladite image de résonance magnétique moyenne.

3. Procédé (10) selon la revendication 2, dans lequel ladite image RM moyenne est fonction d'une moyenne volumique desdites données de RM structurelle et/ou fonctionnelle.

4. Procédé (10) selon l'une quelconque des revendications 1 à 3, dans lequel ledit traitement séparé comprend la génération d'une pluralité de cartes d'activation CF et d'une pluralité de cartes d'activation CS, et lesdites sorties combinées comprennent une concaténation entre une carte d'activation CF respective et une carte d'activation CS respective.

5. Procédé (10) selon l'une quelconque des revendications 1 à 3, dans lequel ledit premier ensemble de couches (41) comprend une couche convolutive cachée (46) entraînée à traiter séparément le CF (20), et une couche convolutive cachée (48) entraînée à traiter séparément le CS (36).

6. Procédé (10) selon l'une quelconque des revendications 1 à 3, dans lequel ledit premier ensemble de couches (41) comprend plus d'une couche convolutive cachée (46) entraînée à traiter séparément le CF (20).

7. Procédé (10) selon l'une quelconque des revendications 1 à 3, dans lequel ledit premier ensemble de couches (41) comprend plus d'une couche convolutive cachée (48) entraînée à traiter séparément le CS (36).

8. Procédé (10) selon l'une quelconque des revendications 1 à 6, dans lequel ledit deuxième ensemble de couches (56) comprend au moins deux couches convolutives cachées (58).

9. Procédé (10) selon l'une quelconque des revendications 1 à 8, dans lequel ladite corrélation est choisie dans le groupe constitué par une corrélation par paires, une corrélation partielle et une corrélation de distance.

10. Procédé (10) selon l'une quelconque des revendications 1 à 9, dans lequel ladite construction dudit CS (36) spécifique au sujet comprend l'application d'une tractographie cérébrale complète pour définir une pluralité de lignes de courant ou de valeurs d'anisotropie fractionnaire entre des paires de régions dudit cerveau, et la conversion de ladite pluralité de lignes de courant ou de valeurs d'anisotropie fractionnaire en une matrice de connectivité, ledit CS (36) spécifique au sujet étant ladite matrice de connectivité.

11. Procédé (10) selon l'une quelconque des revendications 1 à 10, dans lequel ledit CNN (40) est en outre entraîné à prédire une réponse à un traitement de l'état et/ou un résultat clinique de l'état.

12. Procédé (10) selon l'une quelconque des revendications 1 à 11, dans lequel ledit CNN (40) est entraîné à prédire la probabilité d'au moins une affection parmi : commotion cérébrale, trouble dépressif, accident vasculaire cérébral, traumatisme crânien, syndrome de stress post-traumatique, épilepsie, maladie de Parkinson, sclérose en plaques, agitation, maltraitance, maladie d'Alzheimer, anxiété, crise de panique, trouble phobique, trouble bipolaire, trouble de la personnalité limite, trouble du contrôle du comportement, trouble dysmorphique corporel, déficience cognitive, trouble dissociatif, trouble alimentaire, fatigue, trouble du contrôle des impulsions, irritabilité, trouble obsessionnel-compulsif, troubles de la personnalité, trouble psychotique, troubles sexuels, trouble du sommeil, bégaiement, syndrome de Tourette, trichotillomanie, comportement autodestructeur, fibromyalgie, tremblements, schizophrénie, trouble déficitaire de l'attention, trouble d'hyperactivité et troubles d'apprentissage.

13. Produit logiciel, comprenant un support lisible par ordinateur sur lequel sont stockées des instructions de programme, lesquelles, lorsqu'elles sont lues par un processeur de données (122), amènent le processeur de données (122) à recevoir des données de RM fonctionnelle et des données de RM structurelle, décrivant chacune le cerveau d'un sujet, et à mettre en œuvre le procédé (10) selon l'une quelconque des revendications 1 à 12.

14. Système d'imagerie par résonance magnétique (IRM) (100) destiné à l'imagerie du cerveau d'un sujet (102), le système comprenant :
un appareil d'IRM (104, 106, 108) conçu pour effectuer un balayage du cerveau afin de fournir des données de RM fonctionnelle et des données de RM structurelle, chacune décrivant le cerveau, et
un processeur de données (122) conçu pour mettre en œuvre le procédé selon l'une quelconque des revendications 1 à 12.
